(19) Europäisches Patentamt
European Patent Office
Office européen des brevets

(11) Veröffentlichungsnummer: **0 142 653**
**A1**

(12) **EUROPÄISCHE PATENTANMELDUNG**

(21) Anmeldenummer: 84110733.7

(22) Anmeldetag: 08.09.84

(51) Int. Cl.[4]: **C 07 D 249/08**
C 07 D 403/12, C 07 D 233/61
A 01 N 43/653, A 01 N 43/50

(30) Priorität: 22.09.83 DE 3334220

(43) Veröffentlichungstag der Anmeldung:
29.05.85 Patentblatt 85/22

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Schmetzer, Johannes, Dr.
Wacholderweg 45
D-5024 Pulheim(DE)

(72) Erfinder: Stetter, Jörg, Dr.
Gellertweg 4
D-5600 Wuppertal 1(DE)

(72) Erfinder: Reinecke, Paul, Dr.
Lessingstrasse 11
D-5090 Leverkusen 3(DE)

(72) Erfinder: Hänssler, Gerd, Dr.
Heymannstrasse 40
D-5090 Leverkusen 1(DE)

(54) **1-Azolyl-substituierte Oximether.**

(57) Die vorliegende Erfindung betrifft neue 1–azolyl–substituierte Oximether, ein Verfahren zu ihrer Herstellung und ihre Verwendung als Fungizide.

Die Verbindungen der allgemeinen Formel

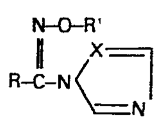

(I)

in welcher

R, R' und X die in der Beschreibung angegebene Bedeutung besitzen,

werden erhalten, wenn man die entsprechenden Oxime mit Alk(en,in)ylhalogeniden gegebenenfalls in Gegenwart eines Lösungsmittels und eines Säurebinders umsetzt.

Die Verbindungen sind gegen Pflanzenkrankheiten in Getreide-, Obst- und Reiskulturen wirksam und eignen sich somit als Pflanzenschutzmittel.

EP 0 142 653 A1

BAYER AKTIENGESELLSCHAFT 5090 Leverkusen, Bayerwerk

Konzernverwaltung RP
Patentabteilung

Slr/Em
(Ia)

# 1-Azolyl-substituierte Oximether

Die vorliegende Erfindung betrifft neue 1-azolyl-substituierte Oximether, ein Verfahren zu ihrer Herstellung und ihre Verwendung als Fungizide.

1-Methylazolyl-substituierte Oximether mit fungizider Wirkung sind bereits bekannt geworden (vgl. dazu DE-OS 2 816 817 /Le A 18 777/, DE-OS 2 723 942 und DE-OS 2 657 578).

Die Wirkung der bekannten Verbindungen ist jedoch insbesondere bei niedrigen Aufwandmengen nicht immer voll befriedigend.

Es wurden nun die neuen 1-azolyl-substituierten Oximether der allgemeinen Formel

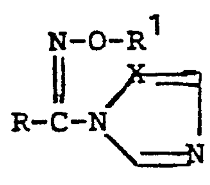

(I)

in welcher

R  für Alkyl, Alkoxyalkyl, Alkenyl, Alkinyl, Cycloalkyl, Aralkyl, Phenoxyalkyl und Phenyl steht, wobei die drei letztgenannten Reste im aromatischen Teil substituiert sein können,

$R^1$  für Alkyl, Alkenyl, Alkinyl, gegebenenfalls substituiertes Aralkyl und Heteroaralkyl steht und

X  für Stickstoff oder die CH-Gruppe steht,

sowie deren physiologisch verträglichen Säureadditionssalze und Metallsalz-Komplexe gefunden.

Man erhält die 1-azolyl-substituierten Oximether der Formel (I), wenn man Oxime der allgemeinen Formel

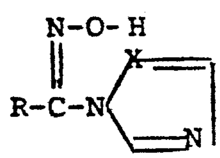

(II)

in welcher
R und X die weiter oben angegebene Bedeutung besitzen,

mit den Alk(en,in)ylhalogeniden der allgemeinen Formel

$$Hal-R^1 \qquad (III)$$

in welcher

$R^1$ die oben angegebene Bedeutung hat und

Hal für Chlor oder Brom steht,

gegebenenfalls in Gegenwart eines Lösungsmittels und eines Säurebinders umsetzt.

Die erfindungsgemäßen Verbindungen der allgemeinen Formel (I) weisen starke fungizide Eigenschaften auf. Überraschenderweise zeigen die erfindungsgemäßen 1-azolyl-substituierten Oximether eine beachtlich höhere fungizide Wirkung als die aus dem Stand der Technik bekannten 1-methylazolylsubstituierten Oximether. Die neuen Verbindungen stellen daher eine Bereicherung der Technik dar.

Die erfindungsgemäßen 1-azolyl-substituierten Oximether sind durch die obige Formel (I) allgemein definiert. Von den Verbindungen der Formel (I) sind bevorzugt diejenigen, bei denen

R für geradkettiges oder verzweigtes Alkyl mit 1 bis 12 Kohlenstoffatomen, für Alkoxyalkyl mit 1 bis 4 Kohlenstoffatomen in jedem Alkylteil, für Alkenyl und Alkinyl mit jeweils 2 bis 5 Kohlenstoffatomen und für Cycloalkyl mit bis zu 6 Kohlenstoffatomen im Ring steht, ferner für Aralkyl mit 1 bis 4 Kohlenstoffatomen im Alkyl- und 6 bis 10 Kohlenstoffatomen im Arylteil steht, wobei der Arylteil gegebenenfalls substituiert sein kann durch Halogen, durch Alkyl- und/oder Alkoxygruppen mit jeweils bis zu 4 Kohlenstoffatomen, und schließlich für Phenoxyalkyl mit

bis zu 3 Kohlenstoffatomen im geradkettigen oder verzweigten Alkylteil und für Phenyl steht, wobei die beiden letztgenannten Reste am Phenyl substituiert sein können durch Halogen, Alkyl- und/oder Alkoxy-Gruppen mit jeweils 1 bis 4 Kohlenstoffatomen,

$R^1$ für Alkyl mit 1 bis 6 und für Alkenyl und Alkinyl mit jeweils 3 bis 5 Kohlenstoffatomen steht, für Aralkyl mit 1 bis3 Kohlenstoffatomen im Alkyl- und 6 bis 10 Kohlenstoffatomen im Arylteil steht, wobei letzterer substituiert sein kann durch Halogen, Alkyl und/oder Alkoxy mit jeweils bis zu 4 Kohlenstoffatomen, und für Heteroaralkyl mit 1 bis 2 Kohlenstoffatomen im Alkylteil und einen aromatischen Heterofünfring mit 1 bis 3 Stickstoffatomen und gegebenenfalls einem Sauerstoffatom im Heteroarylteil steht, und

X die in der Erfindungsdefinition angegebene Bedeutung hat.

Besonders bevorzugt sind diejenigen Verbindungen der Formel (I), bei denen

R für tertiäres Butyl oder Alkoxyalkyl mit 1 bis 3 Kohlenstoffatomen in jedem Alkylteil und für Phenoxyalkyl mit bis zu 3 Kohlenstoffatomen im Alkylteil steht, wobei der Phenylteil ein- bis dreifach durch Fluor, Chlor, Brom, Methyl und/oder Methoxy substituiert sein kann,

$R^1$ für Methyl, Ethyl, Propyl, Butyl, für Allyl,

Propargyl und für Benzyl steht, welches im Phenylteil ein- bis dreifach substituiert sein kann durch Halogen, Methyl und Methoxy, und für Triazolylmethyl und Imidazolylmethyl steht, und

X die in der Erfindungsdefinition angegebene Bedeutung besitzt.

Im einzelnen seien außer den Herstellungsbeispielen die folgenden Verbindungen der allgemeinen Formel

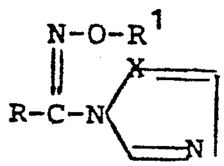

(I)

genannt:

| R | $R^1$ | X |
|---|---|---|
| 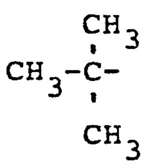 | $CH_3$ | CH |
| " | $C_2H_5$ | " |
| " | $CH_2-CH=CH_2$ | " |
| " | $CH_2-C\equiv CH$ | " |
| " | 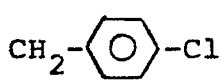 | " |
| " | 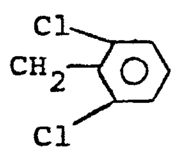 | " |

| R | $R^1$ | X |
|---|---|---|
| 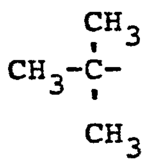 | 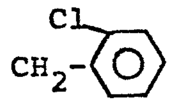 | CH |
| " | 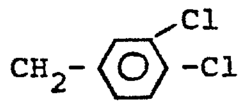 | " |
| " | 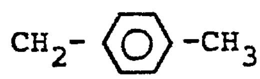 | " |
| " | 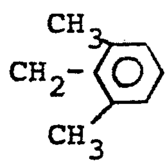 | " |
| " | 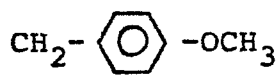 | " |
| " | 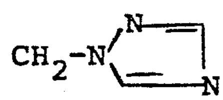 | " |
| " | $CH_3$ | N |
| " | $C_2H_5$ | " |
| " | $CH_2-CH=CH_2$ | " |
| " | $CH_2-C\equiv CH$ | " |

| R | $R^1$ | X |
|---|---|---|
| 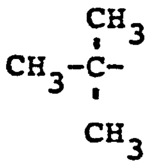 | 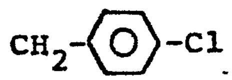 | N |
| " | 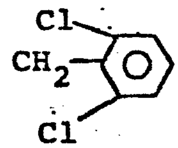 | " |
| " | 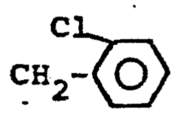 | " |
| " | 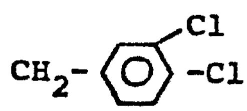 | " |
| " | 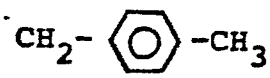 | " |
| " | 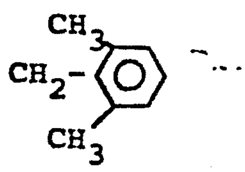 | " |
| " | 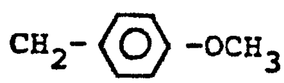 | " |
| " | 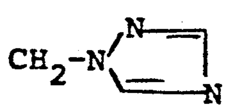 | " |
| 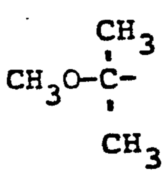 | $CH_3$ | CH |
| " | $C_2H_5$ | " |

| R | $R^1$ | X |
|---|---|---|
| 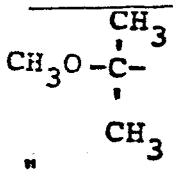 | $CH_2-CH=CH_2$ | CH |
| " | $CH_2-C\equiv CH$ | " |
| " | 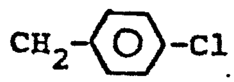 | " |
| " | 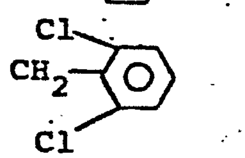 | " |
| " | 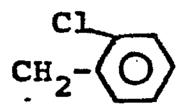 | " |
| " | 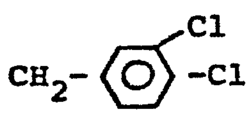 | " |
| " | 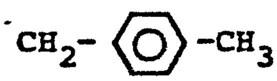 | " |
| " | 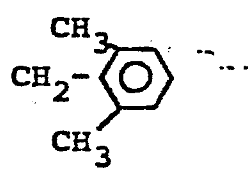 | " |
| " | 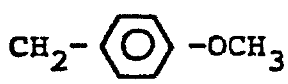 | " |
| " | 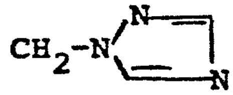 | " |

| R | $R^1$ | X |
|---|---|---|
| 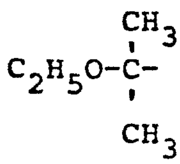 | $CH_3$ | CH |
| " | $C_2H_5$ | " |
| " | $CH_2-CH=CH_2$ | " |
| " | $CH_2-C\equiv CH$ | " |
| " | 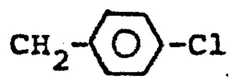 | " |
| " | 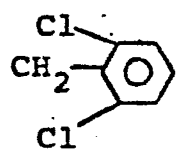 | " |
| " | 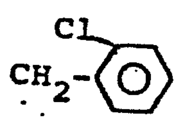 | " |
| " | 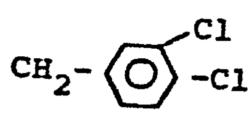 | " |

| R | $R^1$ | X |
|---|---|---|
| 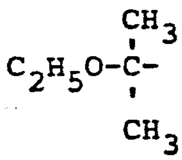 | 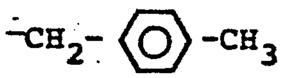 | CH |
| " | 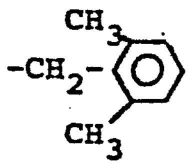 | " |
| " | 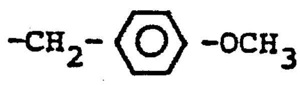 | " |
| " | 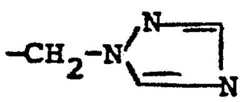 | " |
| 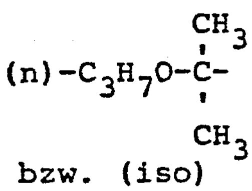 bzw. (iso) | $CH_3$ | " |
| " | $C_2H_5$ | " |
| " | $-CH_2-CH=CH_2$ | " |
| " | $-CH_2-C\equiv CH$ | " |

| R | $R^1$ | X |
| --- | --- | --- |
| $(n)-C_3H_7O-C(CH_3)_2-$ bzw. (iso) | 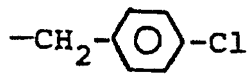 | CH |
| " | 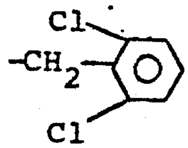 | " |
| " | 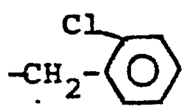 | " |
| " | 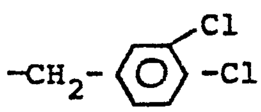 | " |
| " | 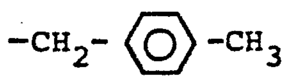 | " |
| " | 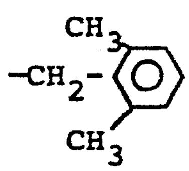 | " |
| " | 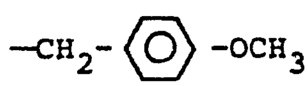 | " |
| " | 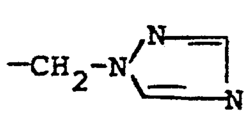 | " |

| R | $R^1$ | X |
|---|---|---|
| 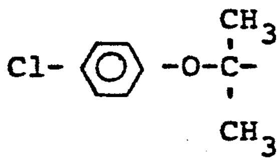 | $CH_3$ | CH |
| " | $C_2H_5$ | " |
| " | $-CH_2-CH=CH_2$ | " |
| " | $-CH_2-C\equiv CH$ | " |
| " | 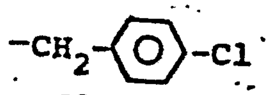 | " |
| " | 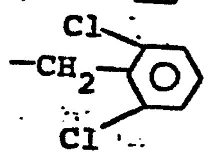 | " |
| " | 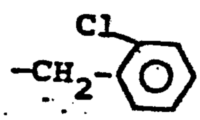 | " |
| " | 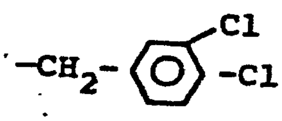 | " |
| " | 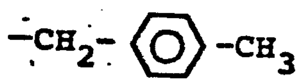 | " |
| " | 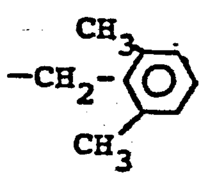 | " |
| " | 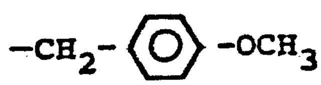 | " |
| " | 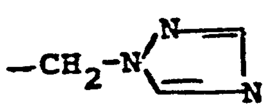 | " |

Verwendet man zur Herstellung der erfindungsgemäßen Verbindungen der Formel (I) beispielsweise 2,2-Dimethyl-1-oximino-1-(N-imidazolyl)-propan und p-Chlorbenzylchlorid als Ausgangsstoffe, so kann der Reaktionsablauf durch das folgende Formelschema wiedergegeben werden:

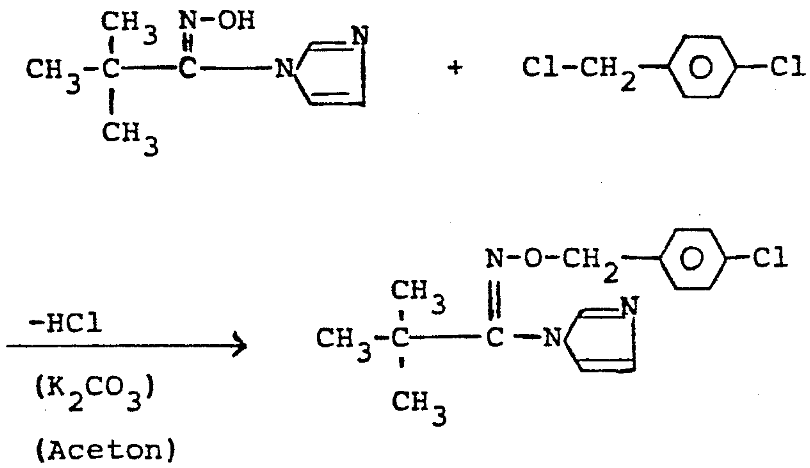


Die erfindungsgemäß als Ausgangsstoffe zu verwendenden Oxime sind durch die allgemeine Formel (II) definiert. In dieser Formel besitzen R und X die weiter oben angegebenen bevorzugten Bedeutungen. Die Verbindungen sind zum Teil bereits bekannt (vgl. dazu die Angaben in der DE-OS 2 613 167 /Le A 17 056/). Sie können zum Beispiel hergestellt werden, indem man Hydroxamsäurehalogenide der Formel

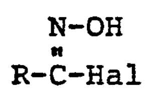

(IV)

in welcher
R die weiter oben angegebene Bedeutung besitzt und
Hal für Chlor oder Brom steht,

mit Azolen der Formel

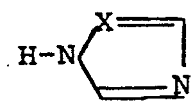

(V)

in welcher
X die oben angegebene Bedeutung besitzt,

in geeigneten organischen Lösungsmitteln, wie z.B. in Ethern wie Tetrahydrofuran oder Dioxan, in Gegenwart eines Säurebinders, wie z.B. Triethylamin, im Temperaturbereich zwischen 0 und +30°C umsetzt. Dabei setzt man zweckmäßigerweise auf 1 Mol der Verbindung der Formel (IV) 1 bis 2 Mole Azol (V) und 1 Mol eines Säurebinders ein. Die Isolierung der Oxime der Formel (II) erfolgt in üblicher Weise durch Abziehen des Lösungsmittels und Ausfällen des Produktes durch Verdünnen mit Wasser.

Die als Vorprodukte verwendeten Hydroxamsäurehalogenide der Formel (IV) sind bekannt (vgl. H. Ulrich "The Chemistry of Imidoyl Halides", Seiten 157-172, Plenum Press, New York (1968) und die dort zitierten Literaturstellen). Noch nicht bekannte Verbindungen lassen sich nach den dort beschriebenen Verfahren leicht herstellen, so z. B. durch Chlorierung der entsprechenden Aldoxime in inerten Lösungsmitteln bei tiefen Temperaturen, z.B. bei -30 bis 0°C.

Neue Hydroxamsäurehalogenide der allgemeinen Formel

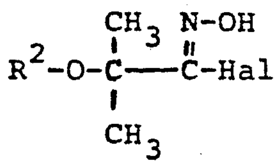

(IVa)

in welcher

$R^2$ für Alkyl und für gegebenenfalls substituiertes Phenyl steht und

Hal für Chlor oder Brom steht,

können hergestellt werden, indem man Halogen-hydroxamsäurehalogenide der Formel

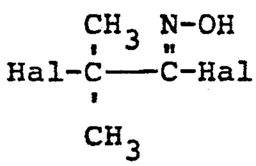

(VI)

in welcher

Hal für Chlor oder Brom steht

in prinzipiell bekannter Weise ("Williamsonsche Ethersynthese") mit Hydroxyverbindungen der Formel

$R^2$-OH (VII)

in welcher $R^2$ die oben angegebene Bedeutung besitzt, zur Umsetzung bringt. Dabei dienen schwach basische Stoffe wie Calciumcarbonat zur Bindung der freiwerdenden Halogenwasserstoffsäure (vgl. dazu die Angaben in J. prakt. Chemie 311, S. 775-785 (1969)). Als Lösungsmittel kann entweder die im Überschuß eingesetzte Hydroxyverbindung oder Acetonitril dienen. Die Umsetzung findet im Temperaturbereich zwischen +50 und 100°C statt. Dabei werden auf 1 Mol der Dihalogenverbindung der Formel (VI) bis zu 10 Mole der Hydroxyverbindung der Formel (VII) und 1 bis 2 Mole Calciumcarbonat eingesetzt. Zur Isolierung der Verbindungen der Formel (IVa) wird das Lösungsmittel abdestilliert und der Rückstand mit Ether extrahiert.

Die Verbindungen der allgemeinen Formel (VI) werden durch Umsetzung von 2 Mol Nitrosylchlorid mit 1 Mol Isobutylen in mit Chlorwasserstoffgesättigten Ether bei Temperaturen unterhalb von 0°C erhalten. (J. prakt. Chemie 311, S. 775-785 (1969)).

Die zur Durchführung der erfindungsgemäßen Umsetzung weiterhin als Ausgangsstoffe benötigten Alk(en,in)ylhalogenide sind durch die allgemeine Formel (III) definiert. In dieser Formel besitzt $R^1$ die weiter oben angegebenen bevorzugten Bedeutungen. Die Verbindungen sind allgemein bekannt. Heteroaralkylhalogenide können beispielsweise so erhalten werden, daß man die entsprechenden Hydroxyverbindungen mit Thionylhalogenid umsetzt (Beilstein H26, E III/IV, S. 46). Die benötigten Hydroxyverbindungen können z.B. erhalten werden, indem man Formaldehyd an ein Azol, wie z.B. 1,2,4-Triazol, addiert (Beilstein H26, E III/IV, S. 326).

Zur Durchführung des erfindungsgemäßen Verfahrens zur Herstellung der 1-azolyl-substituierten Oximether der Formel (I) kommen als Verdünnungsmittel polare organische

Lösungsmittel infrage. Hierzu gehören vorzugsweise Ketone, wie Methylethylketon oder Aceton, ferner Nitrile, wie Acetonitril, Ether, wie Dioxan, und Alkohole, wie Methanol oder Ethanol.

Als Säurebinder können zur Durchführung des erfindungsgemäßen Verfahrens alle üblichen Säurebindungsmittel verwendet werden. Hierzu gehören vorzugsweise Carbonate, wie Natriumcarbonat, Kaliumcarbonat und Natriumhydrogencarbonat, ferner Alkoholate, wie Natriummethylat und Natriumethylat, sowie niedere tertiäre Amine, wie Triethylamin.

Die Reaktionstemperaturen können bei der Durchführung des erfindungsgemäßen Verfahrens in einem größeren Bereich variiert werden. Im allgemeinen arbeitet man zwischen 50 und 150°C, bevorzugt bei der Siedetemperatur des verwendeten Lösungsmittels.

Bei der Durchführung des erfindungsgemäßen Verfahrens setzt man vorzugsweise auf 1 Mol der Verbindung (II) 1 bis 2 Mol der Verbindung (III) und 1 bis 2 Mol eines Säurebinders ein.

Zur Isolierung der Verbindung (I) wird das Lösungsmittel abdestilliert, der Rückstand in Chloroform aufgenommen und mit Natronlauge gewaschen. Die eingeengte organische Phase wird üblicherweise im Hochvakuum von restlichem Lösungsmittel befreit.

Die nach den erfindungsgemäßen Verfahren erhältlichen Verbindungen der Formel (I) können in Säureadditions-Salze bzw. Metallsalz-Komplexe überführt werden.

Zur Herstellung von physiologisch verträglichen Säureadditionssalzen der Verbindungen der Formel (I) kommen vorzugsweise folgende Säuren infrage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.
Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. bis II. sowie IV. bis VIII. Nebengruppe infrage, wobei Kupfer, Zink, Mangan,

Magnesium, Zinn, Eisen und Nickel beispielhaft genannt seien. Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel(I) können in einfacher Weise nach üblichen Verfahren erhalten werden, so z.B. durch Lösen des Metallsalzes in Alkohol, z.B. Ethanol, und Hinzufügen zur Verbindung der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffen zeichnen sich insbesondere durch eine gute fungizide Wirkung gegen Mehltau, Rost, Septoria und Pyrenophora teres an Getreide und gegen Apfelschorf aus. Weiterhin sind die Wirkstoffe gegen Krankheiten im Reisanbau wirksam.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder

Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

## Herstellungsbeispiele

### Beispiel 1

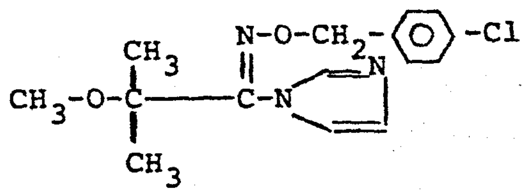

Eine Mischung aus 4,5 g (0,026 Mol) 2,2-Dimethyl-2-methoxy-1-(imidazol-1-yl)-1-oximino-ethan, 4,8 g (0,03 Mol) p-Chlorbenzylchlorid, 3,6 g (0,026 Mol) fein gemahlener Pottasche und 50 ml Aceton wird während 24 Stunden unter Rückflußkühlung zum Sieden erhitzt. Anschließend wird abgesaugt, das Lösungsmittel weitgehend abgezogen und der Rückstand in Chloroform aufgenommen. Die Chloroformphase wird zweimal mit verdünnter Natronlauge gewaschen, über Natriumsulfat getrocknet, und vom Lösungsmittel befreit, zuletzt unter einem Druck von 0,15 mbar, undeiner Heizbadtemperatur von 100°C. Man erhält 4,8 g eines zähen Öles mit dem Brechungsindex $N_D^{20}$= 1,5589, das sind 58 % der Theorie.

Vorprodukt:

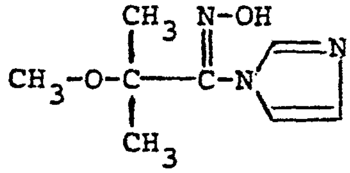

Zu einer Mischung aus 9,0 g (0,132 Mol) Imidazol und 6,7 g (0,066 Mol) Triethylamin in 50 ml absolutem Tetrahydrofuran wird bei 0°C unter Rühren sehr langsam eine Lösung von 9,9 g (0,066 Mol) 2,2-Dimethyl-2-methoxy-1-chlor-1-oximino-ethan zugetropft. Man läßt auf Raumtemperatur kommen und rührt 10 Stunden nach. Der Reaktionsansatz wird filtriert, eingeengt und mit Wasser verdünnt. Das ausgefallene Produkt wird abgesaugt, mit Petrolether gewaschen und getrocknet. Man erhält 10,3 g 2,2-Dimethyl-2-methoxy-1-(imidazol-1-yl)-1-oximino-ethan vom Fp. 155 - 160°C, das sind 85 % der Theorie.

Vorprodukt:

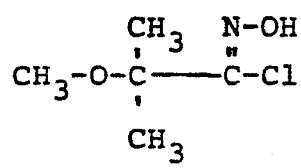

Eine Mischung aus 50 g (0,32 Mol) 2-Methyl-1,2-dichlor-1-oximino-propan, 50 g (0,5 Mol) fein gepulvertem Calciumcarbonat und 500 ml absolutem Methanol wird während 30 Minuten unter Rückflußkühlung zum Sieden erhitzt. Man filtriert ab, engt ein und extrahiert den Rückstand mehrmals mit Ether. Die Etherphase wird über Natriumsulfat getrocknet, filtriert und eingeengt. Man erhält 38,4 g 2,2-Dimethyl-2-methoxy-1-chlor-1-oximino-ethan in Form weißer Kristalle vom Fp. 95°C die Ausbeute beträgt 79 % der Theorie.

Beispiel 2

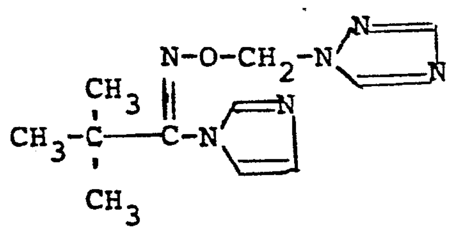

Zu einer Lösung von 3,2 g (0,059 Mol) Natriummethylat in Methanol gibt man langsam 4,6 g (0,029 Mol) Chlor-(1,2,4-triazol-1-yl)-methan-Hydrochlorid. Nachdem die Lösung klar geworden ist, werden noch 5,0 g (0,029 Mol) 2,2-Dimethyl-1-oximino-1-(imidazol-1-yl)-propan zugefügt. Es wird während 10 Stunden bei Raumtemperatur nachgerührt, eingeengt und in sehr verdünnter Sodalösung aufgenommen. Die wäßrige Phase wird mehrmals mit Chloroform extrahiert, über Natriumsulfat getrocknet und anschließend vom Lösungsmittel befreit. Man erhält 3,2 g 2,2-Dimethyl-1-(1,2,4-triazol-1-yl-methyl-oximino)-1-(imidazol-1-yl)-propan als zähes Öl vom Brechungsindex $n_D^{20}$= 1,5152. Die Ausbeute beträgt 45 % der Theorie.

In entsprechender Weise lassen sich die folgenden Verbindungen der allgemeinen Formel

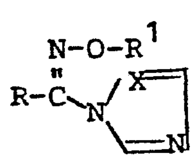

(I)

herstellen:

| Beispiel Nr. | R | $R^1$ | X | Fp (°C) oder $n_D20$ |
|---|---|---|---|---|
| 3 | 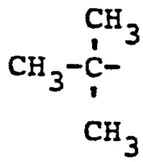 | 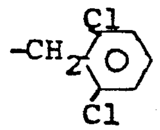 | N | 66 |
| 4 | " | 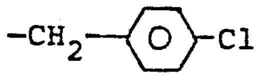 | CH | 1,5465 |
| 5 | " | 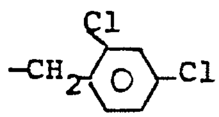 | CH | 1,5692 |
| 6 | 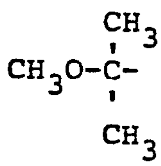 | 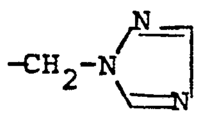 | CH | 1,5130 |
| 7 | " | 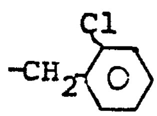 | CH | 1,5671 |
| 8 | " | 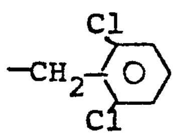 | CH | 1,5775 |
| 9 | 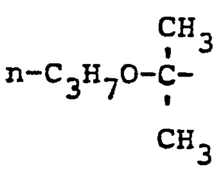 | 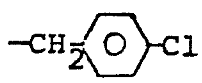 | CH | 1,5450 |
| 10 | 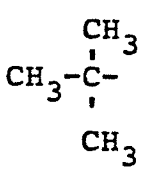 | 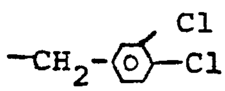 | N | 58 |

| Beispiel Nr. | R | $R^1$ | X | Fp(°C) oder $n_D^{20}$ |
|---|---|---|---|---|
| 11 | 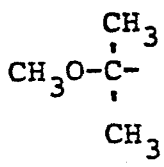 | 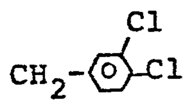 | CH | 1,5620 |
| 12 | 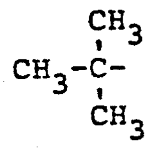 | $CH_3$ | CH | 1,4887 |
| 13 | " | 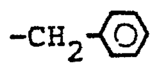 | N | 1,5237 |
| 14 | " | 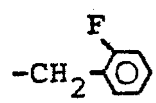 | N | 1,5168 |
| 15 | " | 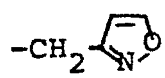 | N | 1,4997 |
| 16 | " | $-CH_2-CH=CH_2$ | N | 1,4820 |
| 17 | " | $-CH_2-C{\equiv}CH$ | N | 60-62 |
| 18 | " | 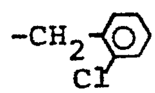 | N | 1,5334 |

Beispiel A:

Cochliobolus sativus-Test (Gerste) /protektiv

Lösungsmittel: 100 Gewichtsteile Dimethylformamid
Emulgator: 0,25 Gewichtsteile Alkylarylpolyglykolether

Zur Herstellung einer zweckmäßigen Wirstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 1, 9, 11.

Beispiel B:

Drechslera graminea-Test (Gerste) / Saatgutbehandlung
(syn. Helminthosporium gramineum)

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von 4°C aus. Dabei wird die Keimung der Gerste und gegebenenfalls auch der Pilzsporen eingeleitet. Anschließend sät man die vorgekeimte Gerste mit 2 x 50 Korn 3 cm tief in eine Standarderde und kultiviert sie im Gewächshaus bei einer Temperatur von ca. 18°C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

Eine deutliche Überlegenheit in der Wirksamkeit gegenüber dem Stand der Technik zeigen in diesem Test z.B. die Verbindungen gemäß folgender Herstellungsbeispiele: 1

Beispiel C:

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 1, 3, 4, 5, 7, 9.

Beispiel D:

Pyricularia-Test (Reis) /systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 1, 3, 10, 4, 7, 9.

Beispiel E

Pellicularia-Test (Reis)

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25°C und 100 % relativer Luftfeuchtigkeit aufgestellt.

5 bis 8 Tage nach der Inokulation erfolgt die Auswertung des Krankheitsbefalles.

Eine deutliche Überlegenheit in der Wirksamkeit gegenüber dem Stand der Technik zeigen bei diesem Test z.B. die Verbindungen gemäß folgender Herstellungsbeispiele: 1, 3, 4, 6, 9.

Patentansprüche:

1) 1-Azolyl-substituierte Oximether der allgemeinen Formel

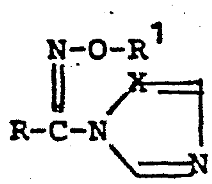

(I)

in welcher

R für Alkyl, Alkoxyalkyl, Alkenyl, Alkinyl, Cycloalkyl, Aralkyl, Phenoxyalkyl und Phenyl steht, wobei die drei letztgenannten Reste im aromatischen Teil substituiert sein können,

$R^1$ für Alkyl, Alkenyl, Alkinyl, gegebenenfalls substituiertes Aralkyl und Heteroaralkyl steht und

X für Stickstoff oder die CH-Gruppe steht,

sowie deren physiologisch verträglichen Säureadditionssalze und Metallsalz-Komplexe.

2) Verbindungen der allgemeinen Formel (I) in Anspruch 1, wobei

R für geradkettiges oder verzweigtes Alkyl mit 1

bis 12 Kohlenstoffatomen, für Alkoxyalkyl mit 1 bis 4 Kohlenstoffatomen in jedem Alkylteil, für Alkenyl und Alkinyl mit jeweils 2 bis 5 Kohlenstoffatomen und für Cycloalkyl mit bis zu 6 Kohlenstoffatomen im Ring steht, ferner für Aralkyl mit 1 bis 4 Kohlenstoffatomen im Alkyl- und 6 bis 10 Kohlenstoffatomen im Arylteil steht, wobei der Arylteil gegebenenfalls substituiert sein kann durch Halogen, durch Alkyl- und/oder Alkoxygruppen mit jeweils bis zu 4 Kohlenstoffatomen, und schließlich für Phenoxyalkyl mit bis zu 3 Kohlenstoffatomen im geradkettigen oder verzweigten Alkylteil und für Phenyl steht, wobei die beiden letztgenannten Reste am Phenyl substituiert sein können durch Halogen, Alkyl- und/oder Alkoxy-Gruppen mit jeweils 1 bis 4 Kohlenstoffatomen,

$R^1$ für Alkyl mit 1 bis 6 und für Alkenyl und Alkinyl mit jeweils 3 bis 5 Kohlenstoffatomen steht, für Aralkyl mit 1 bis 3 Kohlenstoffatomen im Alkyl- und 6 bis 10 Kohlenstoffatomen im Arylteil steht, wobei letzterer substituiert sein kann durch Halogen, Alkyl und/oder Alkoxy mit jeweils bis zu 4 Kohlenstoffatomen, und für Heteroalkyl mit 1 bis 2 Kohlenstoffatomen im Alkylteil und einem aromatischen Heterofünfring mit 1 bis 3 Stickstoffatomen und gegebenenfalls einem Sauerstoffatom im Heteroarylteil steht, und

X die in Anspruch 1 angegebene Bedeutung hat.

3) Verbindungen der Formel (I) in Anspruch 1, wobei

R für tertiäres Butyl oder Alkoxyalkyl mit 1 bis 3 Kohlenstoffatomen in jedem Alkylteil oder für Phenoxyalkyl mit bis zu 3 Kohlenstoffatomen im Alkylteil steht, wobei der Phenylteil ein- bis dreifach durch Fluor, Chlor, Brom, Methyl und/oder Methoxy substituiert sein kann,

$R^1$ für Methyl, Ethyl, Propyl, Butyl, für Allyl, Propargyl und für Benzyl steht, welches im Phenylteil ein- bis dreifach substituiert sein kann durch Halogen, Methyl und Methoxy und für Triazolylmethyl und Imidazolylmethyl steht, und

X die in Anspruch 1 angegebene Bedeutung besitzt.

4) Verfahren zur Herstellung von 1-azolyl-substituierten Oximethern der allgemeinen Formel

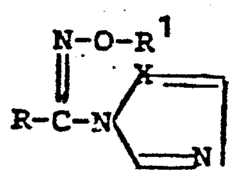

(I)

in welcher

R für Alkyl, Alkoxyalkyl, Alkenyl, Alkinyl, Cycloalkyl, Aralkyl, Phenoxyalkyl und Phenyl steht, wobei die drei letztgenannten Reste im aromatischen Teil substituiert sein können,

$R^1$ für Alkyl, Alkenyl, Alkinyl, gegebenenfalls substituiertes Aralkyl und Heteroaralkyl steht und

X für Stickstoff oder die CH-Gruppe steht,

dadurch gekennzeichnet, daß man Oxime der allgemeinen Formel

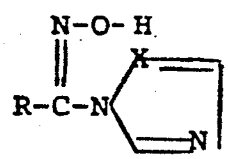

(II)

in welcher

R und X die weiter oben angegebene Bedeutung besitzen,

mit den Alk(en,in)ylhalogeniden der allgemeinen Formel

$$Hal-R^1 \qquad (III)$$

in welcher

$R^1$ die oben angegebene Bedeutung hat und
Hal für Chlor oder Brom steht,

gegebenenfalls in Gegenwart eines Lösungsmittels und eines Säurebinders umsetzt.

5) Fungizide Mittel, gekennzeichnet durch einen Gehalt an mindestens einem 1-azolyl-substituierten Oximether der Formel (I) in Ansprüchen 1 und 4.

6) Verfahren zur Bekämpfung von Pilzen, dadurch gekennzeichnet, daß man 1-azolyl-substituierte Oximether der Formel (I) in Ansprüchen 1 und 4 auf Pilze oder ihren Lebensraum einwirken läßt.

7) Verwendung von 1-azolyl-substituierten Oximethern der Formel (I) in Ansprüchen 1 und 4 als Pflanzenschutzmittel.

8) Verwendung von 1-azolyl-substituierten Oximethern der Formel (I) in Ansprüchen 1 und 4 zur Bekämpfung von Pilzen.

9) Verfahren zur Herstellung von fungiziden Mitteln, dadurch gekennzeichnet, daß man 1-azolyl-substituierte Oximether der Formel (I) in Ansprüchen 1 und 4 mit Streckmitteln und/oder oberflächenaktiven Mitteln vermischt.

0142653

Europäisches Patentamt

# EUROPÄISCHER RECHERCHENBERICHT

Nummer der Anmeldung

EP 84110733.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| --- | --- | --- | --- |
| A | EP - B1 - 0 004 917 (BAYER)<br>* Formel I *<br>-- | 1,5 | C 07 D 249/08<br>C 07 D 403/12<br>C 07 D 233/61<br>A 01 N 43/653<br>A 01 N 43/50 |
| P,A | EP - A3 - 0 095 677 (BASF)<br>* Zusammenfassung *<br>-- | 1 | |
| A | EP - A1 - 0 038 972 (BASF)<br>* Zusammenfassung *<br>-- | 1 | |
| A | DE - A1 - 3 005 899 (HOECHST)<br>* Formel I *<br>-- | 1,5 | |
| A | DE - A1 - 3 150 984 (BAYER)<br>* Formel I *<br>-- | 1,5 | RECHERCHIERTE SACHGEBIETE (Int. Cl.4) |
| A | EP - A1 - 0 018 943 (CIBA-GEIGY)<br>* Zusammenfassung *<br>---- | 1,5 | C 07 D 249/00<br>C 07 D 403/00<br>C 07 D 233/00 |

Der vorliegende Recherchenbericht wurde für alle Patentansprüche erstellt.

| Recherchenort | Abschlußdatum der Recherche | Prüfer |
| --- | --- | --- |
| WIEN | 07-12-1984 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82